# EUROPEAN PATENT APPLICATION

(11) **EP 1 351 058 A1**
(43) Date of publication of application: **08.10.2003**
(21) Application number: 02290819.8
(22) Date of filing: 03.04.2002
(51) Int. Cl.: G01N 33/68

(54) **Fluorescence-based mucus assay**

(71) Applicant: Warner-Lambert Company LLC, Morris Plains, New Jersey 07950 (US)
(72) Inventor: Bader, Thomas, 75014 Paris (FR); Fernandes, Brigitte, 75013 Paris (FR); Henry, Nathalie, 94370 Sucy en Brie (FR)
(74) Representative: Becker, Philippe

(57) **Abstract**

The present invention relates to methods of screening compounds that modulate mucus secretion. The invention also includes compositions, products and kits for use in performing the above methods, as well as the compounds identified by said methods, and their uses. The invention is particularly adapted for (high throughput) screening of various compounds in parallel.

## Description

### Field of Invention

The present invention relates to methods of screening compounds that modulate mucus secretion. The invention also includes compositions, products and kits for use in performing the above methods, as well as the compounds identified by said methods, and their uses. The invention is particularly adapted for (high throughput) screening of various compounds in parallel.

### Background of the Invention

In the therapeutic areas of asthma and COPD, an important endpoint is to quantify the production of mucus. Indeed, during development of these conditions, it is known that mucus production increases, covering the epithelial surface of the respiratory tract thereby reducing normal and proper functioning of the respiratory tract.

Mucus is a fluid comprising water and various macromolecules, including glycoproteins. These glycoproteins are collectively referred to as mucins, and exhibit a molecular weight ranging from few kDa to about 350 kDa. Glycosylation represents the most important part of mucins, as compared to the amino acid portion thereof, and comprises both O-linked and N-linked oligosaccharides. Mucus is produced and secreted by cells of the respiratory epithelium and, as indicated above, mucus hypersecretion is known to participate in the development of asthma and other respiratory diseases such as bronchitis, by narrowing the airway lumen. Mucus secretion is thus an important mechanism that leads to the development and complications of various respiratory diseases, including asthma and COPD, and compounds having the ability to modulate (e.g., to reduce) mucus production or secretion could be of high value in the treatment or prevention of such diseases.

In this respect, several approaches have been envisioned to measure mucus secretion or production, using ELISA assay, phenol-adducts, incorporation of radioactivity or fluorescent derivatives. All of these approaches, however, have limitations, in terms of sensitivity, reproducibility, scaling-up, safety, etc.

The most developed approach is a fluorescent-based assay, which was described for general carbohydrate measurements and was designed to perform a small number of high volume samples in reaction tubes. In particular, Yeadon et al. (Pulm. Pharm. 8 (1995)) reported the measurement of O-linked glycosylated proteins from tracheal secretions by a process involving liberation of oligosaccharides with dilute alkali and subsequent derivatization with 2-cyanoacetamide, to produce fluorescent compounds. However, the method is applicable to large volumes only (around 5 ml), requires various steps, and is not suitable for screening of several compounds.

Accordingly, there is a need in the prior art for methods of measuring or quantifying mucus secretion, that are efficient, sensitive and suitable for scaling up. In particular, there is a need for such methods that are miniaturized, allow increased sample number to be processed, and are amenable to automation.

### Summary of the Invention

The present invention discloses compositions and methods for the screening of compounds that modulate (e.g., inhibit or stimulate) mucus secretion, with reliability and efficacy. The methods according to this invention are simple, reliable, sensitive, convenient and economical, and allow screening of compounds, on a high throughput basis. In particular, the invention can be used to screen, simultaneously, large numbers of compounds, including combinatorial libraries of compounds, to identify drug candidates or targets. The invention allows, for the first time, to screen compounds that inhibit or reduce mucus secretion in airway epithelium, for the selection, improvement and/or development of therapeutically active products.

An object of this invention resides more specifically in a method of measuring mucus secretion in a small volume sample. This method comprises measuring mucus secretion in a sample, the method comprising (i) treating a sample of less than about 500 µl, said sample containing mucus, under conditions suitable to prepare a conjugate of mucus O-linked glycoproteins with an amino-coupling agent and allow to functionalize specifically o-glycosylated mucins, (ii) revealing functionalization by reacting the treated sample with compounds able to fluoresce under appropriate conditions and (iii) measuring fluorescence.
In a specific embodiment of the invention the said sample containing mucus, is prepared under conditions as described above and in step (ii) the treated sample is reacted with a solution of borate and (iii) the fluorescence is measured.

Typically, the method comprises (i) treating the small volume sample to prepare a conjugate of mucus O-linked glycoproteins with 2-cyanoacetamide, (ii) reacting the treated sample with sodium borate to produce fluorescent compounds and (iii) measuring their fluorescence.

Typically, the conjugate of mucus O-linked glycoproteins with amino-coupling agent is prepared by treating the sample, such as 2-cyanoacetamide, with a dilute alkali at elevated temperature. In a specific embodiment, the sample is treated with a solution of CNA at between 0.5 and 0.7M in sodium hydroxide at between 0.1 and 0.2M for about 30 minutes to 1 hour at a temperature comprised between 90 and 95°C.
The treatment reduces O-linked carbohydrates, which then conjugate with 2-cyanoacetamide. Because of the small volumes involved, no cooling step is needed. The small volume of the mucus sample has a volume of less than about 150 µl, preferably of less than about 100 µl, even more preferably below 75µl or 50 µl. Therefore, the method of the invention is performed in 12-, 24-, 48-, or 96- well plates.
In a typical embodiment, the method of the invention is comprising (i) treating a mucus sample of about 25 µl in the presence of about 30µl of a 2-cyanoacetamide solution in sodium hydroxide (1:6) for about 30 to 45 minutes at 90 to 95°C, and (ii) reacting the treated sample with about 200 µl sodium borate (preferably at below 0.3 M) to produce fluorescent compounds. The fluorescence excitation ranges from about 300 to about 350 nm, and wherein emission is from about 385 to about 425 nm.

The sample may be any sample comprising mucus or a dilution or concentrate thereof. The sample may be a cell supernatant, a biological fluid (e.g., broncho alveolar lavage), extra-cellular medium for instance collected from the apical surface of a cell culture, a standard solution, etc. The sample may be pre-treated, to increase purity, increase or decrease concentration, reduce the volumes, etc.

Another object of this invention resides in a method of selecting or identifying a compound that modulates mucus secretion, particularly that inhibits mucus secretion. The method comprises (i) mixing or incubating a test compound with a cell that produces mucus, (ii) collecting a sample of the extracellular medium of said cell and (iii) measuring mucus secretion in said sample, preferably using a method as described above.

Typically, the method includes a step of comparing mucus secretion in the presence and absence of the test compound, or with any reference or standard situation. The cell that produces mucus is typically an epithelial cell such as pulmonary epithelial cell, tracheal epithelial cell, bronchial epithelial cell particularly of human or rodent origin. The cell may be mixed in vivo with the test compound, such as by tracheal administration to an animal and collection of the fluids (e.g., BAL). The compounds are preferably incubated with the cells in culture in vitro, followed by recovery of the cell supernatant (or a portion thereof). The test is particularly suited to identify, select, characterize or optimize compounds that inhibit (e.g., at least partly reduce, decrease) mucus secretion. The test is advantageously carried out in multi well supports, for instance microplates such as several test compounds may be tested in parallel.

Another object of this invention is a method of identifying, selecting, testing, designing or screening a compound that modulates mucus secretion, particularly that inhibits mucus secretion.
In a typical embodiment, the invention is a method of identifying, selecting, characterizing, designing or optimizing the activity of a compound that modulates mucus secretion, comprising (i) mixing in vitro a test compound with an epithelial cell culture that produces mucus, wherein said epithelial cell culture is cultured on a membrane or other suitable device to produce a cell culture having a basal surface and an apical surface, (ii) collecting a sample of the extra-cellular medium of said cell, (iii) measuring mucus secretion in said sample, and optionally, (iv) comparing said mucus secretion measured in the presence of said test compound to that measured in the absence of said test compound to select a compound that decreases mucus secretion.
The method may be used to design a compound that modulate mucus secretion, particularly inhibits mucus secretion, comprising a step of determining whether the compound modulates mucus secretion according to a method previously described and a step of synthesizing or producing said compound that modulates mucus secretion.

A further object of this invention is a method of manufacturing a pharmaceutical composition (particularly for treatment of respiratory diseases), said composition comprising a compound that modulates mucus secretion, particularly that inhibits mucus secretion.

Another object of this invention resides in a kit for use in screening modulators of mucus secretion, the kit comprising a multi-well support and the reagents to perform mucus measurement.

A further object of this invention resides in the use of compounds selected or identified using the above methods, for pharmaceutical, therapeutic or experimental purposes, in particular for the manufacture of a pharmaceutical composition for use in a method of treatment, diagnosis or surgery of the human or animal.

### Legend to the Drawings

Figure 1: Rat tracheal epithelial cells grown on a membrane to support organotypic air-liquid interface cultures. The mucus-secreting cells contain dark stained granules visible in cells on the apical surface of the epithelium.
Figure 2: Test of the influence of different borate concentrations in the reaction mixture.
Figure 3: Comparison between sample determinations done in tubes versus titer plates in the presence or absence of borate during sample derivatisation.
Figure 4: Validation of sample dilutions done in a 96 well titer plate using a multipipetter and PBS.
Figure 5: Test of different filter pairs to optimize the sensitivity of the assay.
Figure 6: Sensitivity and specificity of the fluorescence assay.
Figure 7: Mucus determination using RTE cells supernatants

### Detailed Description of the Invention

As indicated, this invention resides, generally, in improved methods of screening for modulators of mucus secretion. These methods use a miniaturized format, which is adapted for screening large numbers of compounds. In particular, the inventors have now shown that it is possible to detect and measure mucus secretion in sample volumes below 50 µl. The inventors have determined the conditions under which such detection/measuring was feasible, and showed that the assay is sensitive and reproducible. The invention thus enables, for the first time, a screening of the effect of any test compound on this critical aspect of respiratory diseases, thereby allowing selection, identification and manufacture of novel drug candidates or pharmaceutical compounds. Preferably, inhibitors are selected, i.e., compounds that decrease the levels of mucus secretion, typically by at least 20%, preferably by at least 50% as compared to a normal or reference situation (e.g., in the absence of test compound).

An object of this invention resides more specifically in a method of measuring mucus secretion in a sample, the method comprising (i) measuring mucus secretion in a sample, the method comprising (i) treating a sample of less than about 500 µl, said sample containing mucus, under conditions suitable to prepare a conjugate of mucus O-linked glycoproteins with an amino-coupling agent and allow to functionalize specifically o-glycosylated mucins, (ii) revealing functionalization by reacting the treated sample with compounds able to fluoresce under appropriate conditions and (iii) measuring fluorescence.
In a specific embodiment of the invention the said sample containing mucus, is prepared under conditions as described above and in step (ii) the treated sample is reacted with a solution of borate and (iii) the fluorescence is measured.

Typically, the method comprises (i) treating the small volume sample to prepare a conjugate of mucus O-linked glycoproteins with 2-cyanoacetamide, (ii) reacting the treated sample with sodium borate to produce fluorescent compounds and (iii) measuring their fluorescence.

The fluorescence emitted is directly proportional to the amount of mucins in the sample, which is a clear parameter of mucus levels.

The invention particularly stems from the discovery that such a fluorescence-based assay can be performed using very small volume samples of mucus. In particular, all prior art attempts to measure mucus were made in large volume samples, e.g., samples of more than 5 ml. These assays are clearly not suitable for high throughput screening. Also, they are not economical. The invention now shows that high throughput determination of mucus in a sample is feasible. The invention shows that a fluorescence-based assay can be performed on small volume samples of (or containing) mucus. The invention now discloses particular conditions that allow such a method to be carried out.

In particular, because small samples are used, various technical difficulties had to be overcome. One difficulty resides in the fact that the samples were heated at elevated temperatures, which would result in significant evaporation of the sample and possible deterioration thereof. The inventors have now determined that heating is feasible, without altering the reproducibility of the method. Another problem was that in prior experiments, the assay had been performed in large glass tubes which do not suffer from heating. The inventors have now shown that the assay can be performed in other types of supports, such as plastic microtiter plates. The inventors have determined conditions under which heating can be performed without substantially altering the shape of the support. Also, prior experiments lead to a significant precipitation during the reaction. While such a precipitation would not present a problem with large volumes, this is incompatible with small volumes and would have clearly discouraged the skilled person from the instant method. Indeed, the precipitate in a small volume and in a microtitration plate would clearly hamper signal detection (laser excitation and fluorescence detection). The inventors have now shown that conditions can be met to avoid significant precipitation.

The invention thus demonstrates that efficient high throughput mucus secretion is feasible and provides a significant advantage in drug screening and development.

As indicated, the invention uses typically a sample of (or containing) mucus having a volume of less than about 500 µl. Most preferably, the sample of (or containing) mucus has a volume of less than about 400 µl, even more preferably of less than about 250 µl. In most preferred embodiments, the sample of (or containing) mucus has a volume of less than about 150 µl, preferably of less than about 100 µl, even more preferably below 75 µl or 50 µl. The examples show that sample of (or containing) mucus having a volume of about 25 µl can be efficiently processed to measure mucus content. These results are thus particularly surprising and allow the simultaneous (or parallel) processing of many different samples.
In this regards, the invention is well adapted to mucus determination from small quantities of samples such as those obtained from normal or K.O. mice.

The sample may be a cell supernatant, any biological fluid, a standard solution, etc. In a preferred embodiment, the sample is a supernatant of a culture of cells producing mucus. The cells may be for instance epithelial cells, such as most preferably pulmonary epithelial cells. The cells may be treated to produce mucus or to have a stimulated or increased mucus production. The sample may also be a biological fluid, such as a Broncho Alveolar Lavage (BAL), collected from organs or tissues (lung, intestine) that produce mucus under various patho-physiological conditions.

In addition, the sample may be pre-treated, to increase purity, increase or decrease concentration, reduce the volumes, etc.

As indicated, the sample is first treated under conditions suitable to prepare a conjugate of mucus O-linked glycoproteins with an amino-coupling agent such as 2-cyanoacetamide. This reaction allows functionalizing specifically O-glycosylated mucins, which can then be revealed using fluorescent compounds. This reaction is based on β-elimination of the O-linked oligosaccharides, which allows their coupling with reactive agents such as cyanoacetamides or cyanoethanolamides, most preferably, 2-cyanoacetamide (CNA). The chemical reaction has been described in Crowther et al. (Anal. Biochem. 163 (1987) 170).

Typically, the conjugate of mucus O-linked glycoproteins with an amino-coupling agent is prepared by treating the sample, for instance the 2-cyanoacetamide (CNA), with a dilute alkali at elevated temperature. The treatment reduces O-linked carbohydrates which then conjugate with an amino-coupling agent such as 2-cyanoacetamide.

Most particular conditions for the reaction use a solution of CNA in sodium hydroxide, in a volume of less than about 500 µl. The solution of CNA used has a volume that is similar to the volume of the sample containing mucus. Similar means, preferably, that both samples have the same volume or a volume that differs by less than about 25% from each other. In a most preferred embodiment, the sample and the CNA solution have a volume below 200 µl, more preferably below 150 µl, even more preferably below 100 µl. In a specific mode of the invention, the combined volume of the sample and of CNA solution is below 100 µl. As illustrated in the examples, a particular assay condition uses a 25 µl mucus sample and a 30 µl CNA sample.

The CNA sample is more preferably below molar concentration, typically between 0.5 and 1M, more preferably between 0.5 and 0.7M. Optimal results are obtained at 0.6M. The CNA solution is diluted in an alkali, preferably sodium hydroxide, at a concentration below 0.5M. Most preferred NaOH concentration is between, 0.1 and 0.2M, for instance about 0.16M. The ratio CNA:NaOH can be adjusted by the skilled person. In a preferred embodiment, the ratio is comprised between 1:10 and 1:2, for instance around 1:6.

The reaction is carried out at elevated temperature. In a preferred embodiment, the reaction is carried out at a temperature between 85 and 100°C, most preferably below 100°C. The temperature is preferably between about 85 and 95°C, typically between about 90 and 95°C. Indeed, the temperature has to be adjusted to avoid significant evaporation, to avoid alteration of the support (e.g., plastic plate) and still to allow the elimination to occur. The inventors have now found that the reaction can be performed reproducibly by treating the sample for about 30 minutes to 1 hour at a temperature comprised between 90 and 95°C. Under these conditions, a significant β-elimination occurs and a significant CNA coupling takes place, essentially without significantly altering the support or reducing the volumes. Heating can be accomplished using various techniques. However, it is particularly preferred not to use microwaves but a regular oven.

In a second step, the treated sample is reacted with a solution of borate such as sodium borate to produce fluorescent compounds.

It should be noted that, in contrast with prior experiments, no cooling step is needed. Directly upon completion of the first step, the samples can be subjected to sodium borate reaction without delay. Accordingly, the process is simpler. Also, there is no need to use various supports or containers. All reactions are carried out in the same device, which reduces manipulations and enhances efficiency.

The amount of sodium borate added has also been adjusted by the inventors to fulfill the high throughput requirements. In a most preferred embodiment, a solution of less than 500 µl is added, typically of less than 250 µl. The concentration is adjusted by the skilled person to maintain stoechiometry. Typically, a solution of borate at below 0.5M is used, more preferably below 0.3M. In a preferred embodiment, about 200 µl of a 0.1M sodium borate solution is used. It should be understood that borate solutions other then sodium borate might be used.

As illustrated in the examples, a particular assay condition uses a 25 µl mucus sample, a 30 µl CNA sample and a 190 µl borate sample. Under such conditions, the total volume of the whole process does not exceed 245 µl.

In a most preferred embodiment, the reaction is performed by (i) treating a mucus sample of about 25 µl in the presence of about 30 µl of a 2-cyanoacetamide solution in sodium hydroxide (1:6) for about 30 to 45 minutes at 90 to 95°C, and (ii) reacting the treated sample with about 200 µl sodium borate to produce fluorescent compounds.

As indicated, the reaction is preferably performed in multi-wells supports, such as microtitration plates. A suitable format is a 12-, 24-, 48-, 96- or 384-wells plate.

In step (iii), fluorescence can be measured using various techniques known in the art. In a typical embodiment, fluorescence is measured using a fluorescence spectrophotometer that is commercially available (e.g. Wallac VICTOR² 1420 from PerkinElmer, Fluoroscan Ascent FL from Labsystems). Excitation and emission can be performed at various wave lengths or using various filters. Excitation ranges from about 300 to about 350 nm, while emission is from about 385 to about 425 nm. These parameters may be adjusted by the skilled artisan, following the guidelines contained in the present application. In a preferred embodiment, a filter pair of 335/405 nm is used. As illustrated in the examples, these parameters lead to an increased sensitivity and signal to noise ratio.

The fluorescence emitted is directly proportional to the amount of mucins in the sample, which is a reliable parameter of mucus levels in said sample. Indeed, as indicated above, mucin is the major constituent of the mucus. Specifically measuring O-linked oligosaccharides provides direct assessment of mucus level in a sample. The invention now demonstrates that efficient high throughput mucus secretion detection is feasible and provides a significant advantage in drug screening and development.

In this regard, another object of this invention resides in a method of selecting, identifying, designing, optimizing or characterizing a compound that modulates mucus secretion (or production, particularly that inhibits mucus secretion).

The method comprises (i) mixing a test compound with a cell that produces mucus, preferably an epithelial cell that produces mucus, (ii) collecting a sample of the extracellular medium of said cell and (iii) measuring mucus secretion in said sample, preferably using a method as described above.

It should be understood that the term "mucus secretion" designates any step in the whole process of mucus secretion, including without limitation mucus synthesis, production and release.

Typically, the method includes a step of comparing mucus secretion in the presence and absence of the test compound, or with any reference or standard situation. In this regards, it may be useful to have stimulated mucus secretion prior to test a compound in order to analyze its inhibitory activity for instance.

A particular method of this invention is directed at selecting or identifying a compound that inhibits mucus secretion, and comprises (i) contacting or mixing a test compound with a cell that produces mucus, preferably an epithelial cell that produces mucus, (ii) collecting a sample of the extra-cellular medium of said cell, (iii) measuring mucus secretion in said sample, preferably as described above and (iv) comparing said mucus secretion measured in the presence of said test compound to that measured in the absence of said test compound to select a compound that decreases mucus secretion.

Another object of this invention is a method of selecting, identifying, designing, testing, characterizing a compound that modulates mucus secretion particularly that inhibits mucus secretion and/or optimizing its activity. The method comprising (i) mixing in vitro a test compound with an epithelial cell culture that produces mucus, wherein said epithelial cell culture is cultured on a membrane or other suitable device to produce a cell culture having a basal surface and an apical surface, (ii) collecting a sample of the extra-cellular medium of said cell, (iii) measuring mucus secretion in said sample, and optionally, (iv) comparing said mucus secretion measured in the presence of said test compound to that measured in the absence of said test compound to select a compound that decreases mucus secretion.

A further object of the invention is a method of designing a compound that modulates mucus secretion, particularly that inhibits mucus secretion, comprising a step of determining whether the compound modulates mucus secretion according to methods previously described and a step of synthesizing or producing said compound that modulates mucus secretion.

Another object of this invention is a method of manufacturing or optimizing a pharmaceutical composition (particularly for treatment of respiratory diseases), said composition comprising a compound that modulates mucus secretion, particularly that inhibits mucus secretion. The method comprises a step of determining whether the compound modulates mucus secretion, as disclosed above, and a step of formulating said compound or a derivative thereof with a pharmaceutically acceptable diluent or excipient. The method may comprise an intermediate step of synthesizing or producing said compound.

A further object of this invention resides in the use of compounds selected or identified using the above methods, for pharmaceutical, therapeutic or experimental purposes, in particular for the manufacture of a pharmaceutical composition for use in a method of treatment, diagnosis or surgery of the human or animal. An other aspect of this invention is a method of treating a subject comprising administering to a subject in need thereof an effective amount of a compound or composition as defined above. The subject is preferably a human subject, typically having respiratory and/or inflammatory diseases, such as asthma or COPD.

### Mucus-Producing Cells

The effect of the test compound may be assessed in vitro or in vivo. In particular, in step (i), the test compound may be mixed with cells in vitro or in vivo, or in any artificial system that mimics mucus production.

The cells are typically epithelial cells, as discussed below. They may be cultured in vitro, ex vivo, or used as synthetic epithelia. They may be incubated directly in vivo.

In hence, in a first embodiment, the test compound is mixed with cells in vivo, such as by direct administration to an animal so that the compound is placed in contact with the cells producing mucus, preferably with an epithelium. In a particular variant, the test compound is administered in the respiratory airway (e.g., intra-tracheal), so that the compound is in contact with the respiratory epithelium. This can be made with various non-human mammals, including more particularly rodents (rats, mice, etc.). This system is advantageous in that the compounds are assayed in a system that is close to the physiological conditions.
Collection of a sample of the extra-cellular medium of said cells then comprises, for instance, collection of the fluids that surrounds the contacted epithelial cells in the animal, typically collection of broncho-alveolar lavage. This can be performed using known techniques.
The cells may be cells that naturally produce mucus, and/or may be treated first to cause, stimulate or increase mucus production. Increased mucus production is particularly advantageous for selecting inhibitors of mucus production. Indeed, in such case, it is best to have elevated starting levels of mucus secretion. Treatments to cause, stimulate or increase mucus production include the use of various pharmacological agents or treatments, such as (i) pro-inflammatory compounds (e.g., cytokines (such as TNF-α, IL-4, etc. and chemokines), (ii) LPS, prostaglandins, (iii) toxins, (iv) secretion agonists such as ATP etc. In addition (or in the alternative), the cells may be treated with various compounds (smoke, pollution...) to stimulate particular signaling pathways or cellular mechanisms that are particularly targeted. In this regard, phosphodiesterase activators (such as forskolin) may be added to the cell culture.

In a second embodiment, the test compound is incubated with cells in vitro. In this embodiment, cells are cultured in vitro or ex vivo in any appropriate system that allows mucus secretion. The cells may be primary cells or established cell cultures. They may be cultured in any appropriate medium or device.

Typical examples of suitable cells are epithelial cells, particularly mammalian epithelial cells or cell lines and primary cells such as tracheal epithelial cells, bronchial epithelial cells, particularly of human or rodent origin, including the RTE cells and the NHBE cells. Other cells that secrete mucus can be used, even if their phenotype differs from epithelial cells. These include NIH-H292 cells (ATTC# CRL-1848). Other cells are available from culture collections or can be isolated or prepared by conventional techniques.

Furthermore, the cells may be cells that naturally produce mucus, and/or may be treated first to cause, stimulate or increase mucus production. Increased mucus production is particularly advantageous for selecting inhibitors of mucus production. Indeed, in such case, it is best to have elevated starting levels of mucus secretion. Treatments to cause, stimulate or increase mucus production include the use of various pharmacological agents or treatments, such as those above mentioned.
The methods comprising the in vitro mixing or incubating of the cells with the test compound are preferred. Such methods are simpler to run, less expensive and as accurate as those using animals. It should be understood that other methods might be used, using ex vivo tissues or organs, organoïds, and the like.

Upon mixing the cells with the compound, the cell supernatant (or an aliquot thereof) can be recovered.

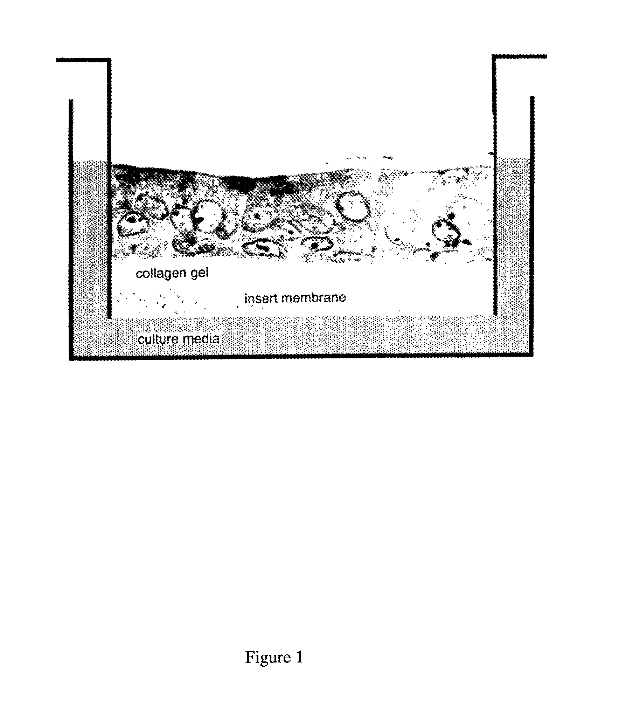

It should be noted that because epithelium are polarized tissues having a basal (or basolateral) surface, that is in contact with the interior of the organism, and an apical surface, that is in contact with the environment, in a most preferred embodiment, this polarity is reproduced (or at least artificially simulated). In this regard, the epithelial cells are preferably cultured on membranes, such that the basolateral surface is in contact with a culture medium and that the apical surface is air-exposed. Such a culture system is disclosed in Example 1 and Figure 1 (see also Guzman K. et al., Am J Physiol. 1996 May 270 (5 Pt 1): L846-53). It should be understood that any other system might be used as well, such as cultures on porous devices and the like.

In that case, the extracellular medium is collected from the apical surface of the culture. Typically, a buffer solution is added to rinse the epithelium and the removed wash is collected. This washing may be repeated several times to recover as much mucus as possible, and the collected removed washes are then pooled.

The cells in vitro may be cultured in their corresponding culture medium, such as any medium commercially available for culture of mammalian cells. These include DMEM, RPMI, etc with or without specific supplements.

The mixing (incubation) in step (i) can last for various periods of time, which can be adjusted by the operator. Typically, this step lasts up to 24 hours or more, preferably less than 24 hours, more preferably less than 12 hours. Generally, the test compounds are preferably incubated with the epithelial cells for a period of time sufficient to allow an interaction to occur and an effect to be caused.

The collected mucus samples may be used directly to measure mucus content, or they may be stored for later use, for instance by freezing.

### The assay conditions

The assay can be performed in any appropriate support or device, including plate, tube, flask, and the like. Generally, incubating is performed in multi-well plates, allowing multiple assays to be carried out in parallel. Typical supports include microtiter plates, especially the 48-, 96- microtiter plate formats, which are easy to manage and easy to illuminate with conventional excitation. Other formats may also be used, including larger microtiter plates or nanotechnologies. The support is preferably a plastic microtitration plate.

Various samples can be analyzed in parallel, as well as reference or standard solutions.

Depending on the support and test compound, varying amounts of reagents can be used in the assay as described above. Typically, a volume of 25 µl of mucus sample is used for each assay, in a total volume of about 250 µl per well, as described above, more particularly for 96 well format.

It should be understood that the precise respective amounts (or concentration) of reagents and test compounds could be adjusted by the user, following guidance provided in the present application. Furthermore, if necessary, the reagents can be mixed in the presence of additional agents to improve the performance of the assay.

A preferred embodiment of this invention includes a method of selecting or identifying a compound that inhibits mucus secretion, and comprises (i) mixing in vitro a test compound with an epithelial cell culture that produces mucus, (ii) collecting a sample of the extra-cellular medium of said cell, (iii) measuring mucus secretion in said sample as described above and (iv) comparing said mucus secretion measured in the presence of said test compound to that measured in the absence of said test compound to select a compound that decreases mucus secretion.

A further preferred embodiment of this invention includes a method of identifying, selecting, characterizing, designing or optimizing a compound that inhibits mucus secretion, and comprises (i) mixing in vitro a test compound with an epithelial cell culture that produces mucus, wherein said epithelial cell culture is cultured in a medium comprising a pro-inflammatory compound, (ii) collecting a sample of the extra-cellular medium of said cell, (iii) measuring mucus secretion in said sample by any suitable method, preferably using a method as described above and optionally (iv) comparing said mucus secretion measured in the presence of said test compound to that measured in the absence of said test compound to select a compound that decreases mucus secretion.

According to further preferred embodiments, the epithelial cells are cultured on a membrane to produce a cell culture having a basal surface and an apical surface, and/or they are of human or rodent origin.

In this regard, a particular embodiment of this invention relates to a method of identifying, selecting, characterizing, designing or optimizing a compound that modulates mucus secretion, comprising (i) mixing in vitro a test compound with an epithelial cell culture that produces mucus, wherein said epithelial cell culture is cultured on a membrane or other suitable device to produce a cell culture having a basal surface and an apical surface, and wherein said culture is preferably performed in a medium comprising a pro-inflammatory compound, (ii) collecting a sample of the extra-cellular medium of said cell, (iii) measuring mucus secretion in said sample, and optionally, (iv) comparing said mucus secretion measured in the presence of said test compound to that measured in the absence of said test compound to select a compound that decreases mucus secretion.

Typically, in step (iii), mucus secretion is measured by treating said sample to produce conjugates of mucus O-linked glycoproteins with a reactive group that can be measured. The reactive group may be directly or indirectly measurable. Specific examples include labeled molecules or molecules that produce a label upon reaction with a further agent. A specific example is 2-cyanoacetamide that reacts with borate to produce fluorescent compounds. Most preferably, mucus secretion is performed using a particularly advantageous method as described in the first part of this application.

### The test (or candidate) compound(s)

The test compound can be any product in isolated form or in mixture with any other material (e.g., any other product(s)). The compound may be defined in terms of structure and/or composition, or it may be undefined. For instance, the compound may be an isolated and structurally-defined product, an isolated product of unknown structure, a mixture of several known and characterized products or an undefined composition comprising one or several products. Examples of such undefined compositions include for instance tissue samples, biological fluids, cell extracts, vegetal preparations, etc. The test compound may be any organic or inorganic product, including a peptide or a polypeptide, a nucleic acid, a lipid, a polysaccharide, a chemical product, or any mixture or derivatives thereof. The compounds may be of natural origin, synthetic origin, including libraries of compounds.
As will be further discussed below, the present invention is particularly adapted for the screening of large numbers of compounds, such as combinatorial libraries of compounds. Indeed, the instant invention provides compositions and methods allowing efficient and simple screening of several compounds in short periods of time. In particular, the instant methods can be partially automated, thereby allowing efficient and simultaneous screening of large sets of compounds.

Generally, the activity of the test compound(s) is unknown, and the method of this invention is used to identify compounds exhibiting the selected property (e.g., mucus secretion modulators). However, in particular instances where the activity (or type of activity) of the test compound(s) is known or expected, the method can be used to further characterize said activity (in terms of specificity, efficacy, etc.) and/or to optimize said activity, by assaying or designing derivatives of said test compounds.

### Uses

The test is particularly suited to identify, select, characterize, test, design or optimize compounds that inhibit (e.g., at least partly reduce) mucus secretion. Such compounds are potential drug candidates for further development or optimization, particularly for treating diseases associated with abnormal mucus secretion, such as respiratory diseases, inflammatory diseases, etc.

A further object of the present invention resides in the use of a compound obtained, identified, selected, tested, designed or characterized as defined above, in the pharmaceutical industry, as a medicament, drug candidate, lead for further optimization, etc. These compounds may for instance be used for the manufacture of a composition for the treatment of the human body, in particular for the treatment of various pathological conditions such as (auto) immune diseases, inflammatory diseases (e.g., chronic obstructive pulmonary disease), allergic diseases such as dermatitis, T helper-1 related diseases, and asthma.

The invention also relates to a pharmaceutical composition comprising a compound obtained, identified, selected or characterized as defined above.

The invention also relates to a method of treating such kinds of diseases in a patient, comprising administering to a patient in need thereof an amount of a compound as described above effective at reducing mucus secretion. Administration can be parenteral or systemic, typically by the respiratory airways.

A further aspect of this invention resides in a method of designing a compound that modulates mucus secretion, particularly that inhibits mucus secretion, comprising a step of determining whether the compound modulates mucus secretion according to the above methods and a step of synthesizing or producing said compound that modulates mucus secretion.

Another object of this application is a method of manufacturing, testing or optimizing a pharmaceutical composition, particularly for treatment of respiratory diseases, said composition comprising a compound that modulates mucus secretion, particularly that inhibits mucus secretion, the method comprising a step of determining whether the compound modulates mucus secretion according to the above methods, and a step of formulating said compound or a derivative thereof with a pharmaceutically acceptable diluent or excipient.

The invention also includes kits for use in screening modulators of mucus secretion. The kit may further include the reagents and/or protocols for screening, such as buffers, etc. A particular kit for use in screening modulators of mucus secretion comprises a multi-well support and the reagents to perform mucus measurement as described above, particularly a CNA and/or a borate preparation, whether in suspension or as a dried or lyophilized material.

Further aspects and advantages of the present invention will be disclosed in the following examples, which should be regarded as illustrative and not limiting the scope of the present application.

### EXAMPLES

### Example 1: Obtaining and culturing rat tracheal epithelial cells (RTE)

This example discloses methods of preparing and culturing rat epithelial cells for subsequent mucus dosage as describe above.

### Day -1: collagen I coated inserts preparation

One day prior to trachea recovery, the collagen I coated inserts are prepared on the evening before seeding the cells. The inserts are coated with 450 µl collagen I each (rat cartilage, Becton Dickinson # 40236, 100 mg in 0.02 N HAc), brought to 3 mg/ml with F-12 medium (Gibco # 21765-029). Then individual inserts are swirled around with forceps or entire plate by careful rocking to spread evenly.
One drop of NH₄(OH) is placed on the cover above each insert, plate is closed and let for col I polymerization.
After col I has gelled in ammonium vapor (∼3 min.) add sterile H₂O to each lower compartment for 10 min. Medium is then aspirated and the operation is repeated once. H₂O is replaced by F-12/Pen-Strep for overnight equilibration. The plates are left in hood overnight.

### Day 0: Trachea recovery and cell isolation

### - Trachea recovery

### Materials for surgery:

PE tubes, about 10 cm long with melted collar, in 70 % EtOH
2-0 silk, about 10 cm long each, stored in 70 % EtOH
2 curved forceps
1 large pair of blunt scissors (8 cm blades)
1 pair of small pointed scissors (3 cm blades)
squirt bottle with 70 % EtOH
one 50 ml falcon tube with ∼15 ml F-12 medium on ice

The tracheal removal is made according to classical methods.

### - Cell isolation (in hood)

### Materials: bulldog clips

Pronase (Sigma protease P-5147), 1 % in F-12, need 1 ml/trachea
6 ml syringe and matching 0.4 micron filter (e.g. Millipore blue)
18-gauge (pink) needle to fit into PE-tubing
small beaker/Falcon tube with F-12 media at room temperature

### Method:

Syringe is filled with pronase and needle is inserted into tube with attached trachea. Trachea is flushed with pronase, then a not is tied at lower end of trachea. Trachea is filled to "banana" extension. Bend tube is attached with syringe and is closed with clip. The needle is removed .
Tube and trachea are hanged in F-12 media in sequence around rim and let digest for exactly 90 min. at room temperature in hood.

### - Preparation of culture medium D9 (or iD9 in case of -HC/-CT)

D9-Medium composition (per 500 ml/0.4µm costar filter with pre-filter):
500 ml DMEM/F-12 (Gibco # 21041-025, with a pinch of extra phenol red). Put about
400 ml in upper filter reservoir and add the following components (in aliquots from -80°C) with BPE last and rinse filter with remaining 100 ml media
500 µl Retinoic Acid (RA) at 5x10⁻⁸ (Sigma # R-2625)
500 µl Insulin (Sigma # I-1882), at 10 mg/ml in 0.01 N HCl
500 µl Transferrin human Apo-TF (Sigma # T-2252), at 5 mg/ml
500 µl mEGF (Collaborative Research [B/D] # 4000) dissolve 100µg vial in 10 ml PBS
500 µl Phosphorylethanolamin (PEA) (Sigma #P-0503) at 706 mg/10 ml PBS
500 µl Ethanolamine (Sigma #E-0135) at 0.5 M (in PBS)
500 µl Choleratoxin (Sigma # C-3012), at 0.1 mg/ml (in PBS)
500 µl Hydrocortisone (Sigma # H-0888), at 2 mg/ml in EtOH, bring to 0.1 mg/ml for the first seven days only
5 ml Pen-Strep (Gibco # 15070-022), is 5000 U/l penicillin, 5 mg/ml streptomycin
5 ml HEPES (Gibco # 15630-056)
10 ml BSA (Sigma # A-7638) in 150 mg/ml, add 6.7 ml Hank's to 10 g BSA
7 ml BPE (Pel Freeze, Rogers, AR)

Rinse filter with remaining ∼100 ml of DMEM/F-12.

### Preparation of culture plates

For the preparation of culture plates (6-well Costar Transwell #3492 [24mm] or #3462 [12-mm]), F-12/Pen-Strep is aspirated from plates and 300 µl (or 80 µl) of FCS and 2 ml (or 0.7 ml) of D9 medium are added to the bottom compartment.

### Cell recovery from tracheas

After 90 min the cells are collected from each trachea in the same order by flushing the tracheas with F-12/Pen-Strep. For this, the needle/syringe is inserted into the tubing, then the bulldog clip is removed and the end of the inflated trachea is almost cut. Then 5 ml are pushed back and forth to dislodge and remove the cells and collect them in a 50 ml Falcon tube. Cell recovery should be approx. 1 Mio. cells/trachea.
The cell suspension is vortexed violently to disrupt cell clusters and the cells are spining down at 800 rpm for 5 min. Then, F-12/Pen-Strep is carefully removed. Two thirds of volume are aspirated and remainder is removed with pipette.
The pellet is re-suspended in D9 medium in a volume inferior to the number of wells x 500 µl (or 100 µl).
The cells are counted in hemocytometer (with trypan blue, live cells are clear).
Then, the cell suspension is diluted to give 250,000 cells/ml and plate 500 µl (100 µl) per insert.
Cells are distributed evenly by moving plate in an 8-shaped movement on bench and plates are placed in humid incubator with 5 % CO₂ at 35-37°C.

At Day 1, 3, 5, 6, medium is removed and replaced with fresh D9 without FCS. Top part receives 500 µl (or 100 µl), the bottom 1,5 ml (or 700 µl) per insert.

At Day 7 through 14, at the air-liquid interface, the medium is removed as before but feed only from the bottom. On day 7 the cultures should be confluent and in a monolayer.

### Example 2: Mucus collection and determination from insert cultures

This example discloses methods collecting mucus for subsequent dosage.

Cells were grown on membranes in inserts (in 24 or 12 mm wells) such that the basolateral surface of the cells is in contact with the medium while the apical surface is air-exposed (air-liquid interface) - See Figure 1.
The cells used are selected among:
. Primary rat tracheal epithelial cells (RTE)
. Primary human bronchial epithelial cells (NHBE)
. A human secretory cell line NIH-H292, available from ATCC (N°CRL-1848).

Under such conditions, the secretion of mucus becomes detectable after approximately ten days in culture and continues to increase. A maximal production occurs during days 13-16. The output can be increased by stimulation with pro-inflammatory cytokines, like TNF-α. Typically, tests are performed on day 14 in culture. Mucus is collected before stimulation to establish a baseline for each individual well and once again after treatment. An appropriate culture system has been described in Guzman K et al. (Am J Physiol. 1996 May 270 (5 Pt 1): L846-53).

The collection of mucus is performed in the following way: In each 24mm insert, 500µl of PBS are added and the epithelium is carefully rinsed and the removed washes collected. This is repeated twice and the samples are pooled to give a total volume of approximately 1.5 ml/insert. In 12mm inserts, the PBS volume is reduced to 100µl. It is preferred to collect the mucus in three washes in order to recover >99% of the secretions. These pooled samples are then frozen for further analysis. The collection method does not change for different cell types.

### Example 3: Influence of borate concentration in fluorescence-based mucus assay

The example reports the results of various experiments that evaluate the influence of different borate concentrations in the reaction mixture. As mucin reference served bovine mucin (Sigma M3895).
Sample: with 25 µl of mucin (Sigma) at 1mg/ml in PBS in 96-well plate, 30 µl of CNA (0.6 M cyanoacatamide/0.16 M NaOH, 1:6) are added and the mixture is heated to 95°C for 30 min. Then, 180 µl - 220 µl (increments of 10 µl) of sodium borate (0.1 M) are added.
Fluorescent plate reader: excitation at 335 nm, emission at 405 nm.

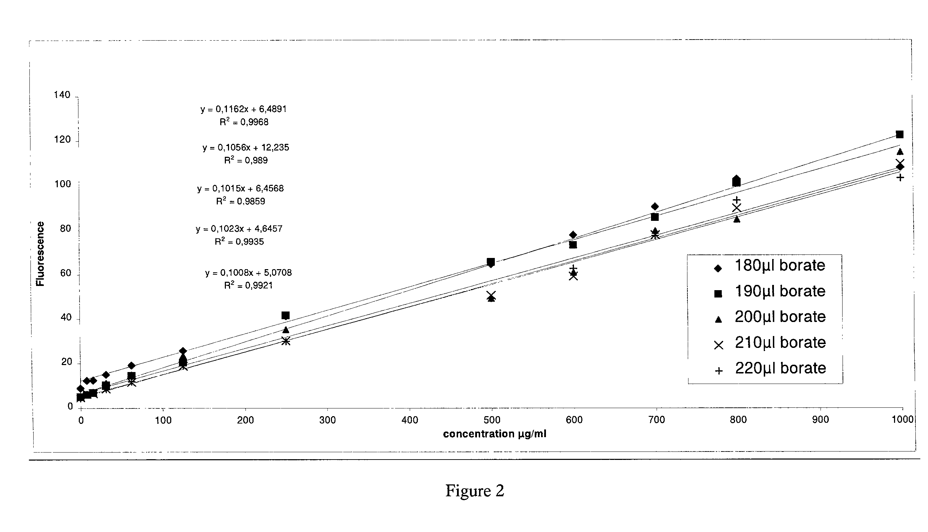

The results are presented Figure 2. According to these results and to avoid precipitations, which obscure the readout in titerplates, the volume of the borate solution was readjusted to 190µl which resulted in an improved sensitivity without precipitation.

### Example 4: Influence of borate addition protocol in fluorescence-based mucus assay

This example discloses a comparison between sample determinations done in tubes versus titer plates in the presence or absence of borate during sample derivatisation.
Sample: 25 µl of mucin (Sigma) at 1mg/ml in PBS in 96-well plate or 250 µl mucin/PBS at 1mg/ml in 5 ml glass tubes.
Then CNA (0.6 M cyanoacatamide/0.16 M NaOH, 1:6) is added: 30 µl to 96-well plate or 300 µl to glass tubes.
Sodium borate (0.1 M) is added in a quantity of 190 µl to half of the 96-wells, 1900 µl to half of the glass tubes.
The reaction mixture is heated to 95°C for 30 min and sodium borate (0.1 M) is added in quantity of 190 µl to the other half of the 96-wells, 1900 µl to the other half of the glass tubes.
Fluorescent plate/tube reader: excitation at 335 nm, emission at 405 nm.

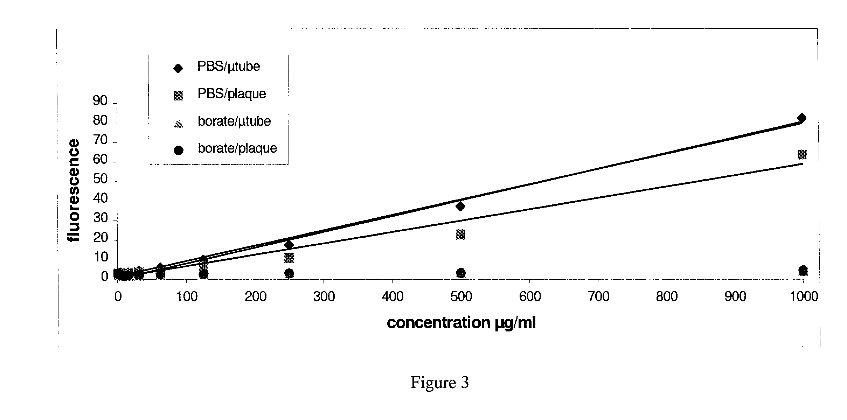

The results are presented in Figure 3. As illustrated, the immediate addition of borate to form the fluorescent complex from the beginning prevents the proper formation thereof. Therefore mucus determination should be performed as a two steps process in which borate is added after the initial NaOH-mediated carbohydrate denaturation.

### Example 5: Fluorescence-based mucus assay in 96 well format

This example demonstrates that the presently disclosed method is suitable for scaling up, particularly in high throughput formats.

Various sample dilutions were deposited in a 96 well titer plate using a multipipetter and PBS. Mucin determination was performed as follows:
Sample: 25 µl of mucin (Sigma) at 1mg/ml in PBS is serially 2-fold diluted directly in 96-well plate. Then, 30 µl of CNA (0.6 M cyanoacatamide/0.16 M NaOH, 1:6) are added to each well. The reaction mixture is heated to 95°C for 30 min. 190 µl of sodium borate (0.1 M) are added to each well.
Fluorescent plate reader: excitation at 335 nm, emission at 405 nm.

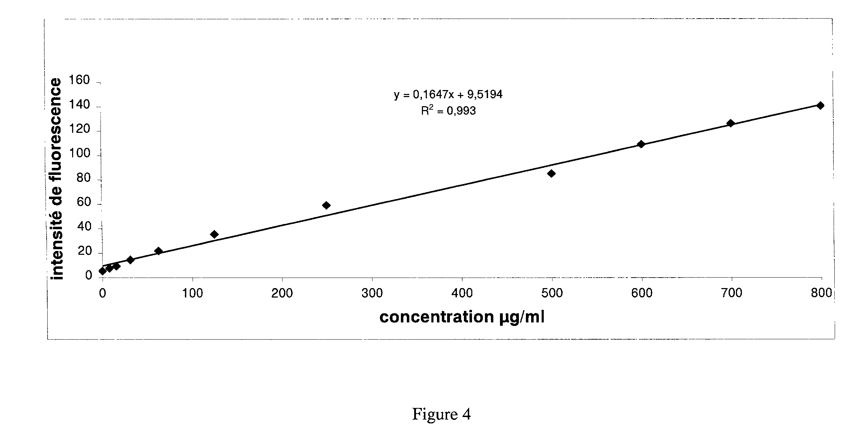

The results are presented Figure 4. The results show that there is no significant adsorption of sample material to the plate which would result in a loss of linearity.

### Example 6: Test of different filter pairs in fluorescence-based mucus assay

This example reports experiments with different filter pairs to increase the sensitivity of the assay.
Sample: standard curve of 25 µl of mucin (Sigma) at 1mg/ml in PBS serially 2-fold diluted and 30 µl of CNA (0.6 M cyanoacatamide/0.16 M NaOH, 1:6) are added to each well. The reaction mixture is heated to 95°C for 30 min. Then, 190 µl of sodium borate (0.1 M) are added to each well.
Fluorescent plate reader settings: excitation at 325 nm, emission at 385 nm.
excitation at 335 nm, emission at 385 nm
excitation at 335 nm, emission at 405 nm

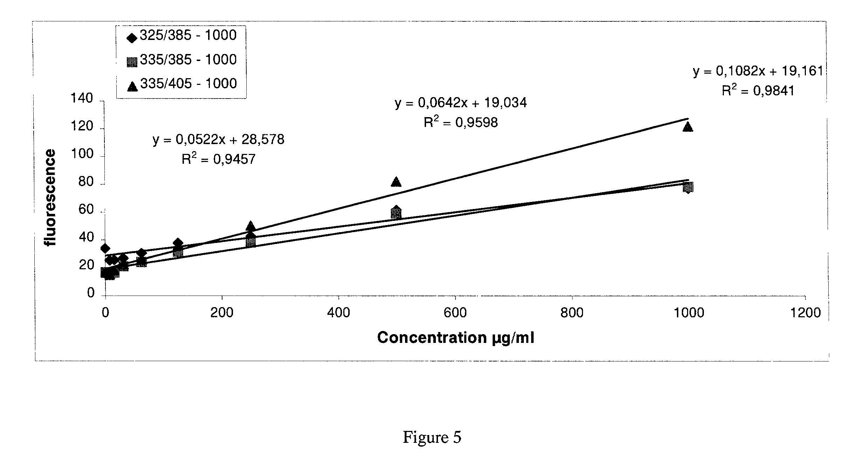

The fluorescent complex, which is formed, can be excited at around 330nm and yields light at around 390nm. Using different combinations of excitation/emission filters (325/385; 335/385 and 335/405, respectively), dose-dependent linear correlations could be established with all three combinations of filters. The results are presented Figure 5. Accordingly, the filter pair 335/405nm showed an overall 30% greater fluorescence and thus a better signal to noise ratio.

### Example 7: Sensitivity and Specificity of the fluorescence-based mucus assay

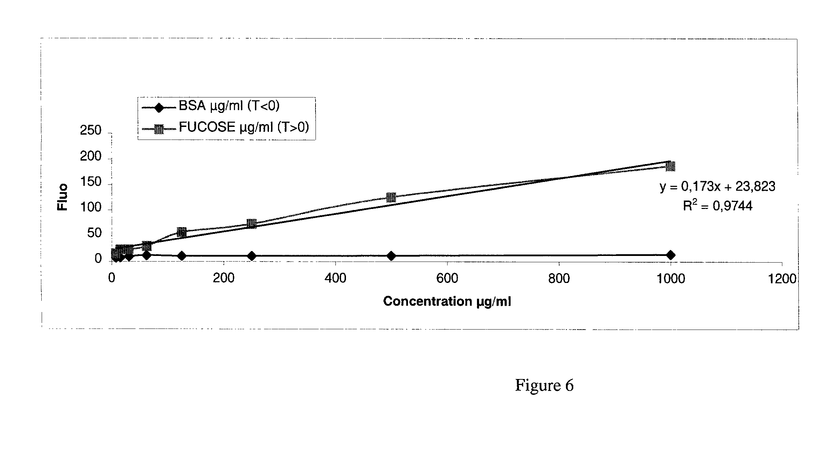

In this experiment the sensitivity and specificity of the fluorescence assay was determined. For that purpose, increasing concentrations of fucose (such as Sigma F8150), as an example for a carbohydrate which is part of mucin, were tested in a 96-well plate.
Samples: 25 µl at 1mg/ml of mucin in PBS is serially 2-fold diluted in 96-well plate.
25 µl at 1mg/ml of fucose in PBS is serially 2-fold diluted in 96-well plate
Then, 30 µl of CNA (0.6 M cyanoacatamide/0.16 M NaOH, 1:6) are added to each well and the mixture is heated to 95°C for 30 min.
190 µl of sodium borate (0.1 M) are added to each well.
Fluorescent plate reader: excitation at 335 nm, emission at 385 nm
The results presented on Figure 6 show that fucose can be detected in a linear range from less than 50 µg/ml. The results also demonstrate that proteins at the same concentration do not interfere in this test.

### Example 8: Fluorescence-based mucus assay in the context of PDE4 inhibitor evaluation

This example discloses a method of selecting, identifying, improving or characterizing compounds with biological activity. The method comprises screening compounds for their ability to modulate (e.g., inhibit) mucus secretion.

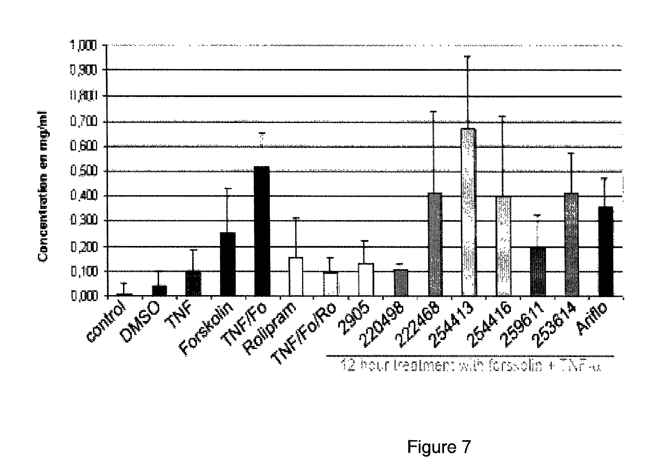

RTE cells are cultured as disclosed in Example 1. The addition of TNF-α into the well serves as an inflammatory challenge, to stimulate mucus secretion. Furthermore, in order to enhance the level of phosphodiesterases, a treatment with forskolin is carried out. The effect of different PDE4 inhibitors on mucus secretion is determined. The results are presented in Figure 7. As demonstrated, the test compounds lead to a different reduction in mucus secretion as compared to the positive control (TNF-α /forskolin) and some approximate the constitutive secretion of unstimulated epithelium (control).

These experiments demonstrate the efficacy and reliability of the presently claimed method.

## Claims

1. A method of measuring mucus secretion in a sample, the method comprising (i) treating a sample of less than about 500 µl, said sample containing mucus, under conditions suitable to prepare a conjugate of mucus O-linked glycoproteins with an amino-coupling agent and allow to functionalize specifically o-glycosylated mucins, (ii) revealing functionalization by reacting the treated sample with compounds able to fluoresce under appriopriate conditions and (iii) measuring fluorescence.

2. A method of measuring mucus secretion in a sample, the method comprising (i) treating a sample of less than about 500 µl, said sample containing mucus, under conditions suitable to prepare a conjugate of mucus O-linked glycoproteins with an amino-coupling agent and allow to functionalize specifically o-glycosylated mucins, (ii) reacting the treated sample with a solution of borate and (iii) measuring fluorescence.

3. A method of measuring mucus secretion in a sample, the method comprising (i) treating a sample of less than about 500 µl, said sample containing mucus, under conditions suitable to prepare a conjugate of mucus O-linked glycoproteins with 2-cyanoacetamide, (ii) reacting the treated sample with sodium borate to produce fluorescent compounds and (iii) measuring fluorescence.

4. The method of any one of claims 1 to 3, wherein the mucus sample has a volume of less than about 150 µl, preferably of less than about 100 µl, even more preferably below 75µl or 50 µl.

5. The method of any one of claims 1 to 4, wherein the sample is a cell supernatant or any biological fluid.

6. The method of claim 4, wherein the sample is a supernatant of an epithelial cell, particularly of a pulmonary epithelial cells.

7. The method of claims 1 to 6, wherein the sample is treated, in step i), in the presence of an amino-coupling agent, with a dilute alkali at elevated temperature.

8. The method of any one of claims 3 to 7, wherein the sample is treated, in step i), in the presence of 2-cyanoacetamide, with a dilute alkali at elevated temperature.

9. The method of claim 8, wherein the sample is treated with a solution of CNA at between 0.5 and 0.7M in sodium hydroxide at between 0.1 and 0.2M.

10. The method of any one of claims 1 to 9, wherein the sample is treated in step i) for about 30 minutes to 1 hour at a temperature comprised between 90 and 95°C.

11. The method of any one of the preceding claims, wherein step ii) comprises treating the sample with a solution of borate at below 0.3M.

12. The method of any one of the preceding claims, comprising (i) treating a mucus sample of about 25 µl in the presence of about 30 µl of a 2-cyanoacetamide solution in sodium hydroxide (1:6) for about 30 to 45 minutes at 90 to 95°C, and (ii) reacting the treated sample with about 200 µl sodium borate to produce fluorescent compounds.

13. The method of any one of the preceding claims, wherein fluorescence excitation ranges from about 300 to about 350 nm, and wherein emission is from about 385 to about 425 nm.

14. The method of any one of the preceding claims, wherein the method is performed in 12-, 24-, 48- or 96- well plates.

15. A method of selecting or identifying a compound that modulates mucus secretion, the method comprising (i) mixing a test compound with a cell that produces mucus, particularly with an epithelial cell that produces mucus, (ii) collecting a sample of the extracellular medium of said cell and (iii) measuring mucus secretion in said sample according to the method of any one of claims 1 to 14.

16. The method of claim 15, further comprising a step of comparing mucus secretion in the presence and absence of the test compound, or with any reference or standard situation.

17. A method of selecting or identifying a compound that inhibits mucus secretion, comprising (i) mixing a test compound with a cell that produces mucus, particularly with an epithelial cell that produces mucus, (ii) collecting a sample of the extra-cellular medium of said cell, (iii) measuring mucus secretion in said sample according to the method of any one of claims 1 to 14, and (iv) comparing said mucus secretion measured in the presence of said test compound to that measured in the absence of said test compound to select a compound that decreases mucus secretion.

18. The method of any one of claims 15 to 17, wherein the test compound is mixed with epithelial cells in vivo, such as by direct administration to an animal so that the compound is placed in contact with an epithelium.

19. The method of any one of claims 15 to 17, wherein the test compound is incubated with epithelial cells in vitro.

20. The method of claim 19, wherein the epithelial cells are mammalian epithelial cells or cell lines, such as tracheal epithelial cells, bronchial epithelial cells, particularly of human or rodent origin.

21. The method of claim 19 or 20, wherein the epithelial cells are cultured on a membrane or any other suitable device, such that the basolateral surface is in contact with a culture medium and that the apical surface is air-exposed.

22. The method of any one of claims 15 to 21, wherein, upon mixing the cells with the compound, the cell supernatant (or a portion thereof) is recovered.

23. The method of claim 21, wherein, upon mixing the cells with the compound, the extracellular medium is collected from the apical surface of the culture.

24. The method of any one of claims 15 to 23, wherein the epithelial cells are cells that naturally produce mucus, or cells treated to stimulate mucus production.

25. The method of any one of claims 15 to 24, wherein the method is performed in a 12-, 24-, 48-, 96- well plates.

26. The method of any one of claims 15 to 25, wherein several test compounds are tested in parallel.

27. A method of identifying, selecting, characterizing, designing or optimizing the activity of a compound that modulates mucus secretion, comprising (i) mixing in vitro a test compound with an epithelial cell culture that produces mucus, wherein said epithelial cell culture is cultured on a membrane or other suitable device to produce a cell culture having a basal surface and an apical surface, (ii) collecting a sample of the extra-cellular medium of said cell, (iii) measuring mucus secretion in said sample, and optionally, (iv) comparing said mucus secretion measured in the presence of said test compound to that measured in the absence of said test compound to select a compound that decreases mucus secretion.

28. A method of designing a compound that modulates mucus secretion, particularly that inhibits mucus secretion, comprising a step of determining whether the compound modulates mucus secretion according to any one of claims 13 to 25 and a step of synthesizing or producing said compound that modulates mucus secretion.

29. A method of manufacturing a pharmaceutical composition, particularly for treatment of respiratory diseases, said composition comprising a compound that modulates mucus secretion, particularly that inhibits mucus secretion, the method comprising a step of determining whether the compound modulates mucus secretion according to any one of claims 13 to 25, and a step of formulating said compound or a derivative thereof with a pharmaceutically acceptable diluent or excipient.

30. Use of compounds selected or identified using the method according to any one of claims 15 to 27 for the manufacture of a medicament or a pharmaceutical composition for use in a method of treatment, diagnosis or surgery of the human or animal, particularly of a respiratory disease, such as asthma or COPD.
